**Europäisches Patentamt**

**European Patent Office**

**Office européen des brevets**

(11) Veröffentlichungsnummer : **0 155 352
B1**

# EUROPÄISCHE PATENTSCHRIFT

(45) Veröffentlichungstag der Patentschrift :
04.05.88

(21) Anmeldenummer : 84111821.9

(22) Anmeldetag : 03.10.84

(51) Int. Cl.⁴ : **A 61 M 16/00**, A 61 M 16/10,
A 61 M 16/16

(54) Beatmungsgerät mit hochfrequenter Beatmung.

(30) Priorität : 20.01.84 DE 3401849

(43) Veröffentlichungstag der Anmeldung :
25.09.85 Patentblatt 85/39

(45) Bekanntmachung des Hinweises auf die Patenterteilung : 04.05.88 Patentblatt 88/18

(84) Benannte Vertragsstaaten :
AT DE FR GB NL SE

(56) Entgegenhaltungen :
EP-A- 0 080 155
US-A- 4 182 599
US-A- 4 322 594

(73) Patentinhaber : Drägerwerk Aktiengesellschaft
Moislinger Allee 53-55
D-2400 Lübeck 1 (DE)

(72) Erfinder : Kühl, Uwe
Redder 43
D-2419 Mustin (DE)
Erfinder : Müller, Eckhardt, Dr. Dipl.-Phys. Dr. rer.nat
1-18-27 Shoto Shibuya-Ku
Tokyo 150 Japan-Nippon (JP)

## Beschreibung

Die Erfindung betrifft ein Beatmungsgerät mit hochfrequenter Beatmung.

Eine wesentliche Frage bei der Hochfrequenzbeatmung (HFJV) stellt sich aus der Beherrschung der Anfeuchtung und der Temperatur des Atemgases. Das dem Patienten zugeführte Gasgemisch besteht aus zwei Teilströmen, dem unter hohem Vordruck durch eine Düse oder Kanüle zugeblasenen Atemgas (Jet-Beatmung) und einem durch Injektorwirkung angesaugten Zusatzgas. Bei der Hochfrequenzbeatmung werden dabei im Atemgasstrom Gasdruckimpulse erzeugt.

Aus DE-A-33 08 819 (veröffentlicht nach dem Prioritätstag) ist ein derartiges Beatmungsgerät mit hochfrequenter Beatmung bekannt. Bei ihm führt eine Atemgasleitung von einer Atemgasquelle über ein durch eine Steuerung geschaltetes Hochfrequenzventil und eine Atemgasheizung zu einer Jet-Mündung in einem Trachealtubus. Für die Befeuchtung sorgt eine Dampferzeugungseinrichtung, der Wasser zugeführt wird. Über eine Dampfleitung ist die Dampferzeugungseinrichtung an die Atemgasleitung zwischen der Atemgasheizung und der Jet-Mündung angeschlossen. Die Dampferzeugungseinrichtung wird von der Steuerung in Abhängigkeit von dem Hochfrequenzventil geschaltet und bläst den erzeugten Dampf in die Atemgasleitung ein.

Aufgabe der Erfindung ist ein Beatmungsgerät mit hochfrequenter Beatmung, ausgerüstet mit einer Befeuchtungseinrichtung für das Atemgas über die Dampfphase dadurch zu verbessern, daß eine einfache Warneinrichtung gegen Störungen der Atemgas- und Dampfzufuhr sowie der Atemgasheizung vorgesehen wird.

Diese Aufgabe wird erfindungsgemäß dadurch gelöst, daß in der Atemgasleitung bei dem Anschluß der Dampfleitung ein Temperaturfühler angeordnet und mit einer Warneinrichtung verbunden ist.

Die mit der Erfindung erzielten Vorteile bestehen insbesondere darin, daß durch die Ausrüstung mit nur einem zusätzlichen Temperaturfühler nebst Warneinrichtung das richtige Arbeiten des Gerätes überwacht werden kann und aus verschiedenen Ursachen auftretende Fehler erkannt werden können. So führen Überfunktion der Dampfzufuhr oder der Atemgasheizung sowie ungenügendes Öffnen des Hochfrequenzventils zu einem Überschreiten der Normaltemperatur, dagegen mangelhaftes Schließen des Hochfrequenzventils sowie Unterfunktion der Dampfzufuhr oder der Atemgasheizung zu deren Unterschreiten. Die Lage des Temperaturfühlers bei dem Anschluß der Dampfleitung an die Atemgasleitung sichert einen geringen Abstand zu den möglichen Fehlerquellen und damit eine sofortige Erkennung auftretender Fehler. Durch die Warneinrichtung, die auch die Auslösung von Sicherheitsschaltungen umfaßt, können Gefahren für den Patienten vermieden und die Untersuchung des Fehlers unverzüglich aufgenommen werden.

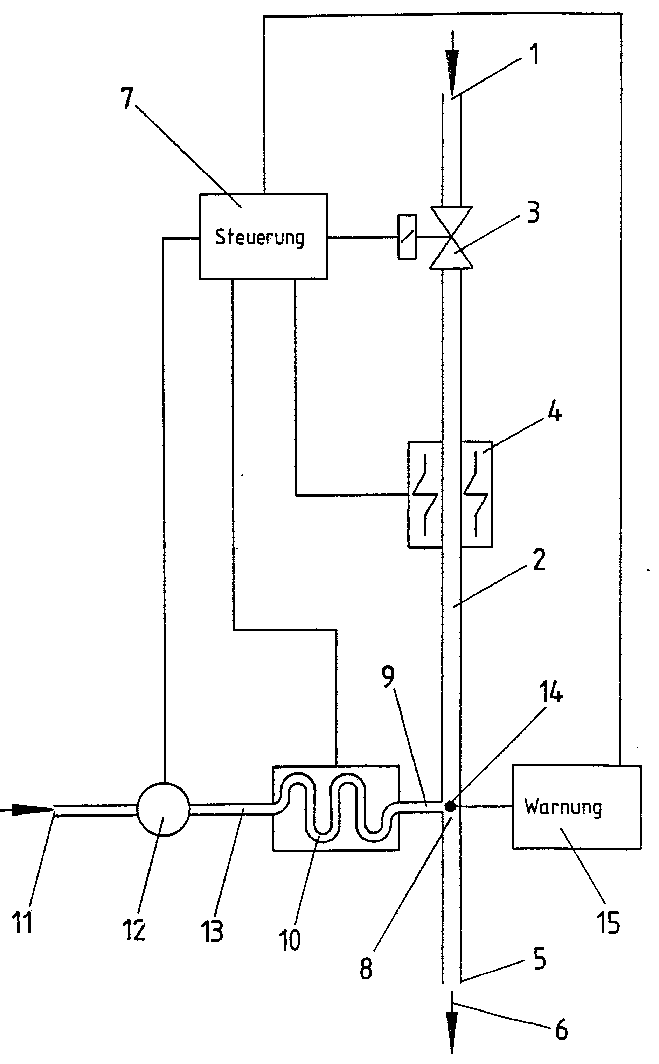

Ein Ausführungsbeispiel der Erfindung ist in der Zeichnung dargestellt und wird im folgenden beschrieben.

Das Beatmungsgerät besteht aus dem in der Zeichnung dargestellten hochfrequenten Hochdruckteil für das Atemgas und dem nicht dargestellten mit der Jet-Mündung 5 verbundenen Zusatzgasteil (Patientensystem).

Das unter Hochdruck stehende Atemgas wird aus einer Atemgasquelle bei 1 in die Atemgasleitung 2 eingeleitet und durchströmt dann ein Hochfrequenzventil 3, eine Atemgasheizung 4 und die Jet-Mündung 5. Hier wird es entspannt und bei 6 in Richtung Patient geblasen.

Die Schaltung des Hochfrequenzventils 3 erfolgt durch die Steuerung 7.

Zwischen der Atemgasheizung 4 und der Jet-Mündung 5 ist bei 8 eine Dampfleitung 9 an die Atemgasleitung 2 angeschlossen, durch die der in der Dampferzeugungseinrichtung erzeugte Wasserdampf, durch die Steuerung 7 in Abhängigkeit von dem Hochfrequenzventil 3 gesteuert, eingeblasen wird.

Die Dampferzeugungseinrichtung besteht aus einer engen von außen beheizten Dampfwendel 10 und einer Dosierpumpe 12. Das zu verdampfende Wasser wird bei 11 zugeführt und von der Dosierpumpe 12, angesteuert von der Steuerung 7, durch eine Wasserleitung 13 in die Dampfwendel 10 gedrückt, dort verdampft und als Wasserdampf durch die Dampfleitung 9 in die Atemgasleitung 2 eingeblasen. Es mischt sich dort dem in der Atemgasheizung 4 aufgewärmten Atemgas zu.

Ein Temperaturfühler 14 ist bei dem Anschluß 8 der Dampfleitung 9 in der Atemgasleitung 2 angeordnet und mit einer Warneinrichtung 15 verbunden. Im Betrieb auftretende Fehler an den vorgeschalteten Elementen, wie Fehlfunktion des Hochfrequenzventils 3, der Atemgasheizung 4 oder der Dosierpumpe 12, wirken sich als Änderungen der Temperatur des Atemgas-Dampf-Gemisches aus. Sie werden durch den Temperaturfühler 14 der Warneinrichtung 15 gemeldet, die bei wesentlichen Abweichungen einen Alarm gibt und Sicherheitsschaltungen an der Steuerung 7 auslöst.

## Patentanspruch

Beatmungsgerät mit hochfrequenter Beatmung durch eine Steuerung (7) über ein Hochfrequenzventil (3) und eine Atemgasheizung (4) in einer Atemgasleitung (2), die als Verbindung zwischen einer Atemgasquelle (1) und einer Jet-Mündung (5) in einem Trachealtubus dient, sowie mit einer von der Steuerung in Abhängigkeit von dem Hochfrequenzventil geschalteten Dampferzeugungseinrichtung (10), die über eine Dampfleitung (9) zwischen der Atemgasheizung und der Jet-Mündung an die Atemgasleitung angeschlossen ist, wobei in der Atemgasleitung (2) bei dem Anschluß (8) der Dampfleitung (9) ein Temperaturfühler (14) angeordnet und mit einer Warneinrich-tung (15) verbunden ist.

**Claim**

Breathing apparatus with high-frequency respiration using a controller (7) by way of a highfrequency value (3) and a respiratory gas heater (4) in a respiratory gas circuit (2), which serves as a connection between a respiratory gas source (1) and a jet-orifice (5) in a tracheal tube, as well as with a steam generating device (10) controlled by the controller in dependence on the high-frequency valve, which device is attached to the respiratory gas circuit between the respiratory gas heater and the jet-orifice by way of a steam line (9), a temperature sensor (14) being arranged in the respiratory gas circuit (2) at its junction (8) with the steam line (9), said sensor being connected to a warning device (15).

**Revendication**

Appareil respiratoire à respiration à haute fréquence au moyen d'un dispositif de commande (7) et par l'intermédiaire d'une valve à haute fréquence (3) et d'un dispositif de chauffage de gaz respiratoire (4) prévu dans une conduite de gaz respiratoire (2) qui sert de liaison entre une source de gaz respiratoire (1) et un embout à tuyère (5) disposé dans un tube trachéal, ainsi qu'avec un dispositif générateur de vapeur (10) qui est mis en marche par le dispositif de commande en fonction de la valve à haute fréquence, et qui est raccordé par l'intermédiaire d'une conduite de vapeur (9) à la conduite de gaz respiratoire entre le dispositif de chauffage de gaz respiratoire et l'embouchure à tuyère, une sonde de température (14), reliée à un dispositif d'avertissement (15), étant disposée dans la conduite de gaz respiratoire (2) au niveau du raccordement (8) de la conduite de vapeur (9).

0 155 352